# EUROPEAN PATENT APPLICATION

(11) **EP 4 647 105 A1**
(43) Date of publication of application: **12.11.2025**
(21) Application number: 24770717.7
(22) Date of filing: 07.03.2024
(51) Int. Cl.: A61M 27/00

(54) **WOUND TREATMENT DEVICE**

(30) Priority: 10.03.2023 JP 2023037584
(71) Applicant: Teikyo University, Tokyo 173-8605 (JP)
(72) Inventor: KAINUMA, Masahiko, Tokyo 173-8605 (JP)
(74) Representative: E. Blum & Co. AG
(86) International application number: PCT/JP2024/008799
(87) International publication number: WO 2024/190611

(57) **Abstract**

A wound treatment device (1) includes: a transfer tube (2) that has one end portion (2a1) which is inserted into a body of a patient and that transfers exudate generated inside the body to an outside of the body; and a manual suction pump (3) that is connected to the other end portion (2b2) of the transfer tube (2) outside the body and that suctions the exudate into an inside thereof through the transfer tube (2), in which the suction pump (3) includes an accommodation part (3a) that creates a negative pressure in the internal space by elastic recovery after deformation due to external pressure, and that suctions and accommodates the exudate in the body into the internal space through the transfer tube (2), a discharge part (3b) that is provided to be openable and closable and that is capable of discharging a fluid in the internal space of the accommodation part (3a), and a check valve (3c) that is provided at a connection location with the transfer tube (2) and that prevents a backflow of the fluid from the internal space of the accommodation part (3a) to the transfer tube (2), a flow path for the exudate is formed in the transfer tube (2), and a first opening portion accessible to the flow path is formed in addition to an end portion opening formed in each of the one end portion (2a1) and the other end portion (2b2) of the transfer tube (2).

## Description

### TECHNICAL FIELD

The present invention relates to a wound treatment device.

Priority is claimed on Japanese Patent Application No. 2023-037584, filed March 10, 2023, the content of which is incorporated herein by reference.

### BACKGROUND ART

In the related art, in the treatment of a wound, generally, a step of infection control is first performed, which includes a treatment such as debridement for opening and washing a wound part and further removing necrotic tissue in some cases. Thereafter, after forming granulation tissue by negative pressure wound therapy that promotes healing of the wound part by suctioning and discharging an exudate exuding from the wound part in an open state, the wound part is epithelialized by secondary wound healing, skin grafting, myocutaneous flap, or the like.

As a device that is used in the negative pressure wound therapy, for example, Patent Document 1 discloses a wound treatment device that can collect the exudate of a wound part in a container through a hose by connecting a tip part of the hose extending from a vacuum pump to a pad and covering the wound part with the pad.

In addition, Patent Document 2 discloses a wound treatment device that can supply a washing solution to a wound part while discharging the exudate exuding from the wound part through a hose.

### Citation List

### Patent Documents

Patent Document 1: Published Japanese Translation No. 2003-521962 of the PCT International Publication
Patent Document 2: Japanese Unexamined Patent Application, First Publication No. 2012-105841

### SUMMARY OF INVENTION

### Technical Problem

However, in a wound treatment device that is used in the negative pressure wound therapy, a vacuum pump or the like capable of exhibiting a strong suction force is adopted as suction means, and thus there is a problem in that a power source is required, and there is a problem in that the weight of the entire device is extremely heavy, the size is bulky, and the movement of the patient is restricted.

The present invention has been made in view of such circumstances, and an object of the present invention is to provide a wound treatment device that does not require a power source and hardly restricts the movement of a patient.

### Solution to Problem

In order to solve the above problems, an aspect of the present invention includes the following aspects.

[1] A wound treatment device includes: a transfer tube that has one end portion which is inserted into a body of a patient and that is configured to transfer an exudate generated inside the body to an outside of the body; and a manual suction pump that is connected to the other end portion of the transfer tube outside the body and that is configured to suction the exudate into an inside thereof through the transfer tube, in which the suction pump includes an accommodation part that creates a negative pressure in the internal space by elastic recovery after deformation due to external pressure, and that suctions and accommodates the exudate in the body into the internal space through the transfer tube, a discharge part that is provided to be openable and closable and that is configured to discharge a fluid in the internal space of the accommodation part, and a check valve that is provided at a connection location with the transfer tube and that is configured to prevent a backflow of the fluid from the internal space of the accommodation part to the transfer tube, a flow path for the exudate is formed inside the transfer tube, connecting the one end portion and the other end portion, and in addition to an end portion opening formed in each of the one end portion and the other end portion, a first opening portion accessible to the flow path is formed in the transfer tube.
[2] In the wound healing device according to the above [1], the transfer tube includes a drain having one end portion that is inserted into the body of the patient, and a relay tube that connects the drain and the suction pump, and the drain is switchable between a state where the drain is connected to the relay tube and a state where the connection with the relay tube is released, and the first opening portion is formed in the other end portion of the drain, which is connected to the relay tube.
[3] In the wound treatment device according to the above [2], the transfer tube includes a connector that connects the drain and the relay tube from an outside.
[4] In the wound treatment device according to any one of the above [1] to [3], the transfer tube has a substantially Y-shaped configuration that branches into two at a portion on the other end portion side with respect to a center portion in an extension direction, one end portion out of two branched end portions is connected to the suction pump, the first opening portion is formed at the other end portion, and a lid covering the first opening portion is detachably attached to the other end portion.
[5] In the wound treatment device according to any one of the above [1] to [3], a substantially tubular covering member is provided on a radially outer side of the transfer tube to cover an outer peripheral surface of a portion of the transfer tube in an extension direction, and the first opening portion radially penetrates the outer peripheral surface of a part of the transfer tube in a circumferential direction, which is covered with the covering member, to the flow path.
[6] In the wound treatment device according to the above [5], the covering member is rotatably provided on the radially outer side of the transfer tube, and a second opening portion that radially penetrates to an inside of the covering member is formed in a part of the covering member in the circumferential direction.
[7] In the wound treatment device according to the above [5], a turned-up portion configured to be turned up away from the outer peripheral surface of the drain is formed in the covering member, and a blocking piece configured to block the first opening portion is provided on an inner surface of the turned-up portion facing the first opening portion.
[8] In the wound treatment device according to the above [3], a third opening portion that radially penetrates to the flow path is formed on an outer peripheral surface of the drain, and an inner peripheral surface of the drain is circular.
[9] A wound treatment kit includes: the wound treatment device according to any one of the above [1] to [8]; and a cleaning tool for cleaning an inside of the transfer tube.
[10] In the wound treatment kit according to the above [9], the cleaning tool is a rod-shaped brush.
[11] In the wound treatment kit according to the above [9], the cleaning tool is a hollow tube connected to a syringe, and an outer diameter of the tube is smaller than an inner diameter of the drain.
[12] The wound treatment kit according to any one of the above [9] to [11] further includes: a transfer tube fixture that includes an adhesive part that is attached to a skin of a patient; and a fixing part that is attached to the adhesive part and that is configured to detachably fix the transfer tube.

### Advantageous Effects of Invention

According to the present invention, it is possible to provide a wound treatment device that hardly restricts the movement of a patient because it can be manufactured to be lightweight and compact without requiring a power source due to using a manual pump.

### BRIEF DESCRIPTION OF DRAWINGS

[FIG. 1] An overall view of a wound treatment device according to a preferred embodiment.
[FIG. 2A] A partially enlarged view of the vicinity of one end portion of a drain as viewed from the direction of an arrow A shown in FIG. 1.
[FIG. 2B] A partially enlarged view of the vicinity of one end portion of the drain as viewed from the direction of an arrow B shown in FIG. 1.
[FIG. 2C] A partially enlarged view of the vicinity of one end portion of the drain as viewed from the direction of an arrow C shown in FIG. 1.
[FIG. 3] A partial longitudinal sectional view showing the state of an inside of a suction pump shown in FIG. 1.
[FIG. 4A] An overall perspective view of a joint.
[FIG. 4B] A schematic perspective view showing a state where the joint is pulled out from the drain and the connection between the drain and a relay tube is released.
[FIG. 5A] A schematic plan view showing an insertion step in a method for treating a wound part using the wound treatment device.
[FIG. 5B] A schematic plan view showing a closing step in the method for treating a wound part using the wound treatment device.
[FIG. 5C] A schematic plan view showing a connection step in the method for treating a wound part using the wound treatment device.
[FIG. 6A] A partially enlarged perspective view showing a state where an inside and an outside of a drain of a wound treatment device according to another preferred embodiment are in communication with each other.
[FIG. 6B] A partially enlarged perspective view showing a state where the inside and the outside of the drain shown in FIG. 6A are isolated from each other.
[FIG. 6C] A partially enlarged perspective view of the drain.
[FIG. 6D] A perspective view of a first covering member that covers the drain.
[FIG. 6E] A partial longitudinal sectional view taken along line W-W shown in FIG. 6A.
[FIG. 6F] A partial longitudinal sectional view taken along line V-V shown in FIG. 6B.
[FIG. 7A] A schematic plan view showing a state where a lid is attached to the other end portion of a branch of a drain of a wound treatment device according to still another preferred embodiment.
[FIG. 7B] A diagram showing a state where the lid is detached from the other end portion of the branch of the drain.
[FIG. 8A] An enlarged plan view of the vicinity of a drain and a second covering member of a wound treatment device according to another preferred embodiment.
[FIG. 8B] An enlarged perspective view of the vicinity of the drain showing a state where a turned-up portion of the second covering member shown in FIG. 8A is greatly turned up.
[FIG. 8C] An enlarged perspective view of the vicinity of the drain showing a state where the turned-up portion of the second covering member shown in FIG. 8A is attracted to the drain.
[FIG. 8D] A partially enlarged perspective view of the drain showing a state where the second covering member is detached.
[FIG. 9A] A schematic perspective view showing a transfer tube fixture included in a wound treatment kit.
[FIG. 9B] A schematic perspective view showing a state where the transfer tube is attached to the transfer tube fixture shown in FIG. 9A.
[FIG. 10] A sectional end view showing a connection state between a drain and a relay tube of a wound treatment device according to still another preferred embodiment.

### DESCRIPTION OF EMBODIMENTS

Hereinafter, preferred embodiments of the present invention will be described in detail with reference to the accompanying drawings.

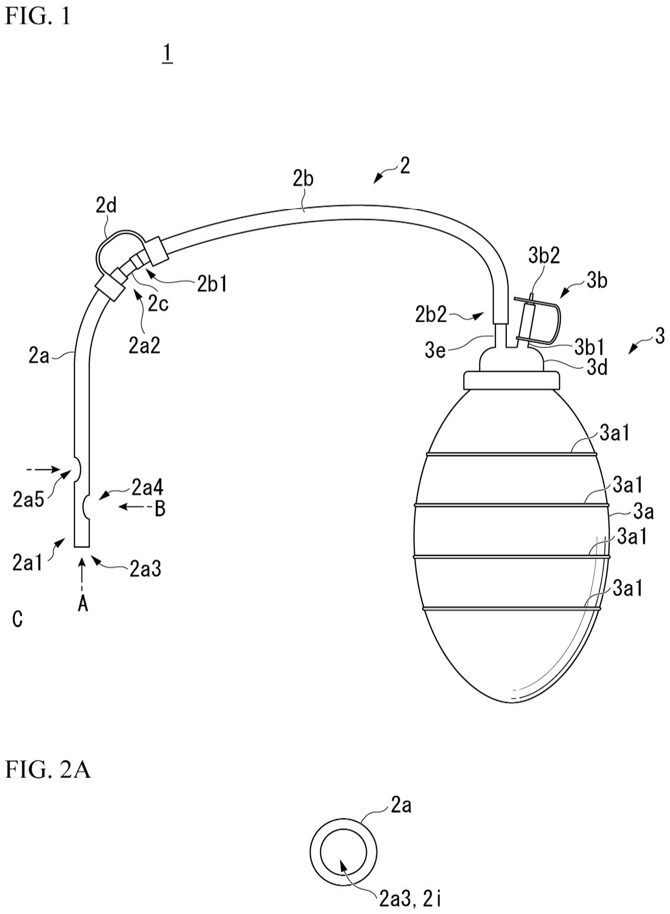

FIG. 1 is an overall view of a wound treatment device 1 according to a preferred embodiment.

The wound treatment device 1 includes a hollow transfer tube 2 that transfers an exudate generated inside the body and located inside a wound part of a patient, a suction pump 3 that suctions the exudate into an inside thereof through the transfer tube 2, and a clip (not shown) that is provided outside the suction pump 3 to be attachable to clothing or the like of the patient and be portable. One end portion of the transfer tube 2 is disposed inside the wound part, and when negative pressure is applied by the suction pump 3 in a state where the wound part is closed, the exudate inside the wound part is discharged to the outside of the body, whereby the healing of the wound part can be effectively accelerated as compared with a treatment method of the related art.

The suction pump 3 is a substantially elongated spherical container having a function of a manual pump that does not require a power source, and can suction and accommodate the exudate into the inside through the transfer tube 2 when the inside is under negative pressure.

The transfer tube 2 includes a drain 2a which is partially inserted into the body of the patient from the one end portion 2a1 side, a relay tube 2b having one end portion 2b1 that is connected to the drain 2a, and the other end portion 2b2 that is connected to the suction pump 3, a connector 2d, and a joint 2c.

In the present specification, with respect to each end portion of the drain 2a and the relay tube 2b, an end portion on the upstream side in a flow path for the exudate will be referred to as one end portion, and an end portion on the downstream side will be referred to as the other end portion.

The drain 2a and the relay tube 2b of the present embodiment each has an outer diameter of 20 Fr (French, approximately 6.7 mm) which is substantially the same, and are connected to each other through the joint 2c to be capable of transferring the exudate.

The drain 2a is switchable between a state where the drain 2a is connected to the relay tube 2b via the joint 2c and a state where the connection to the relay tube 2b is released. That is, the connection between the drain 2a and the relay tube 2b can be released and reconnected.

As will be described in detail later, the cleaning of the inside of the drain 2a with one end portion 2a1 inserted into the body can be performed by releasing the connection between the drain 2a and the relay tube 2b (in other words, by detaching the drain 2a from the relay tube 2b).

The relay tube 2b that connects the drain 2a and the suction pump 3 to each other is formed to be transparent, so that the state of the inside can be seen from the outside. However, the transparency of the relay tube 2b is not particularly limited. Therefore, for example, the relay tube may be formed to be translucent or opaque.

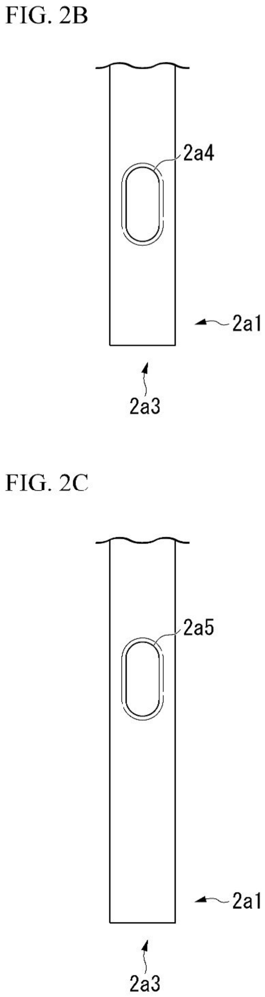

FIG. 2A is a partially enlarged view of the vicinity of one end portion 2a1 of the drain 2a as viewed from the direction of an arrow A shown in FIG. 1, FIG. 2B is a partially enlarged view of the vicinity of one end portion 2a1 of the drain 2a as viewed from the direction of an arrow B shown in FIG. 1, and FIG. 2C is a partially enlarged view of the vicinity of one end portion 2a1 of the drain 2a as viewed from the direction of an arrow C shown in FIG. 1.

A flow path 2i for the exudate, which connects one end portion (one end portion of the drain 2a) 2a1 of the transfer tube 2 and the other end portion (the other end portion of the relay tube 2b) 2b2, is formed inside the transfer tube 2 (refer to FIG. 2A).

On the one end portion 2a1 side with respect to the center portion in an extension direction of the drain 2a, an opening portion 2a3 is formed at one end portion 2a1, and two opening portions 2a4 and 2a5 (third opening portions) are formed in the vicinity of one end portion 2a1. The opening portion 2a4 and the opening portion 2a5 are formed to have different phases from each other in a circumferential direction of the drain 2a.

As shown in FIGS. 2B and 2C, each of the two opening portions 2a4 and 2a5 is formed such that a part of the outer peripheral surface of the drain 2a radially penetrates to the inside of the drain (in other words, to the flow path 2i for the exudate). Each of the two opening portions 2a4 and 2a5 has a substantially elliptical shape and has a width of about 1 cm in the extension direction of the drain 2a (the direction of the flow path).

The opening portion 2a4 is located on the other end portion 2a2 side about 1 cm far from one end portion 2a1, and the opening portion 2a5 is located on the other end portion 2a2 side about 2.5 cm far from one end portion 2a1.

As described above, the opening portions 2a4 and 2a5 are formed on the outer peripheral surface of the drain 2a, so that even in a case where the opening portion 2a3 formed at one end portion 2a1 of the drain 2a is blocked inside the body, the exudate can be continuously suctioned and discharged through the two opening portions 2a4 and 2a5.

The inner diameter of the drain 2a is preferably 2 mm or more in order to prevent internal obstruction, and the outer diameter of the drain 2a is preferably 2.7 mm (8 Fr in terms of outer circumference) or more and 1 cm or less in order to allow natural healing after the drain 2a is pulled out from the inside of the body and to maintain a suction force. However, there is no limitation thereto.

The shape of the inner peripheral surface of the drain 2a (in other words, the shape of the longitudinal cross section of the drain 2a) is preferably a smooth circle, as shown in FIG. 2A. Since the inner peripheral surface of the drain 2a is circular, the inside of the drain 2a does not have corners, so that the inside of the drain 2a can be easily cleaned and negative pressure can be easily applied.

On the other hand, for example, in a case where the shape of the inner peripheral surface is a quadrangle, there is a disadvantage that waste products are accumulated at four corners and negative pressure is difficult to be applied. In this case, waste products of a discharged liquid may accumulate in the drain, which may cause drain infection.

In addition, in a case where a so-called flat drain is used, since a tube 5a of 8 Fr (described in detail later) cannot be inserted into the inside of the drain, the drain is likely to be blocked from a distal end, there is a risk of drain infection, and it is necessary to replace the drain.

In the present embodiment, the drain 2a is formed of a silicone resin, and the relay tube 2b is formed of polyvinyl chloride. However, the materials of the drain 2a and the relay tube 2b are not limited thereto.

As the materials of the drain 2a and the relay tube 2b, a biomaterial having biocompatibility, being sterilizable, having no toxicity, and having durability is desirable. As such a biomaterial, for example, synthetic rubber such as chloroprene, natural rubber, or a polymer material such as polyvinyl chloride, polyethylene, polypropylene, silicone, polyurethane, fluororesin, polyester, or polytetrafluoroethylene can be given.

In addition, it is preferable that the drain 2a and the relay tube 2b are formed of a material having high flexibility. By using a material having high flexibility, damage to an organ, a blood vessel, or the like can be suppressed.

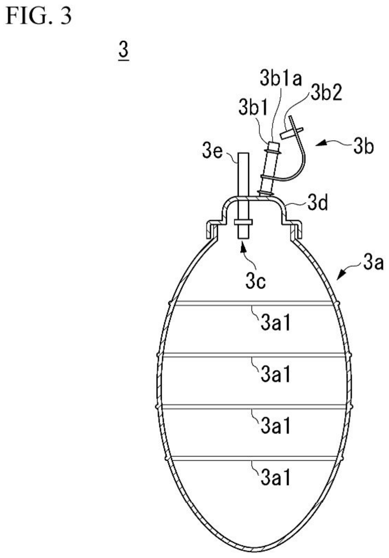

FIG. 3 is a partial longitudinal sectional view showing the state of the inside of the suction pump 3 shown in FIG. 1.

The suction pump 3 includes an accommodation part 3a that accommodates the exudate transferred from the inside of the body through the transfer tube 2 in the inside thereof, a discharge part 3b for discharging a fluid in the accommodation part 3a to the outside of the suction pump 3, a check valve 3c that prevents a backflow of the fluid from the internal space of the accommodation part 3a to the transfer tube 2, a lid part 3d that covers an upper portion of the accommodation part 3a, and a connection tube 3e that is connected to the other end portion 2b2 of the relay tube 2b. The connection tube 3e and the discharge part 3b are formed to protrude substantially upward from the lid part 3d. The fluid as referred to herein specifically refers to air or an exudate.

A scale 3a1 indicating the volume of the exudate inside the accommodation part 3a is formed on the outer side surface of the accommodation part 3a, and the volume of the exudate accommodated inside the accommodation part 3a can be confirmed by forming the accommodation part 3a to be transparent or translucent.

The lid part 3d, the connection tube 3e, and the check valve 3c are formed of a translucent biomaterial.

The connection tube 3e has an outer diameter slightly larger than the inner diameter of the relay tube 2b, and the relay tube 2b can be connected to the connection tube 3e by inserting the connection tube 3e into the relay tube 2b from the other end portion 2a2 side of the relay tube 2b having flexibility.

The check valve 3c is provided at a connection location with the transfer tube 2. More specifically, the check valve 3c is connected to the connection tube 3e from below, and a space inside the check valve 3c communicates with a space inside the connection tube 3e. While the accommodation part 3a is under negative pressure, the check valve 3c is opened to cause the space inside the accommodation part 3a to communicate with the inside of the connection tube 3e and the inside of the transfer tube 2.

The discharge part 3b includes a discharge tube 3b1 having an inside protruding substantially upward, and a plug 3b2 that is detachably inserted into the discharge tube 3b1. The discharge tube 3b1 is formed of resin, and the plug 3b2 is formed of a rubber material.

The plug 3b2 can be inserted into and removed from the discharge tube 3b1 from a discharge port 3bla at an upper end of the discharge tube 3bl. As shown in FIG. 1, in a state where the plug 3b2 is inserted into the discharge tube 3b1, the fluid inside the accommodation part 3a cannot be discharged to the outside through the discharge port 3bla. As shown in FIG. 3, the fluid inside the accommodation part 3a can be discharged from the discharge port 3b1a to the outside of the suction pump 3 only after the plug 3b2 is detached from the discharge port 3b1a.

Hereinafter, a state where the plug 3b2 is detached from the discharge tube 3b1 and the fluid inside the accommodation part 3a can be discharged is referred to as a "discharge state", and a state where the plug 3b2 is inserted into the discharge tube 3b1 and the fluid inside the accommodation part 3a cannot be discharged is referred to as a "non-discharge state".

A base portion of the plug 3b2 is attached to the outer peripheral surface of the discharge tube 3b1, and thus there is no risk of losing the plug 3b2 even when the plug 3b2 is detached from the discharge tube 3b1.

The accommodation part 3a is formed of silicone resin having elasticity, and in a case where the accommodation part 3a is pressed from the outside by an operator in the discharge state where the plug 3b2 is detached, the fluid inside the accommodation part 3a is discharged from the discharge port 3b1a by external pressure, and the accommodation part 3a is deformed and compressed. Hereinafter, a state where the accommodation part 3a is pressed from the outside is referred to as a "pressed state", and a state where the accommodation part 3a is not pressed from the outside is referred to as a "non-pressed state".

In the non-discharge state, since there is no escape route for the fluid inside the accommodation part 3a, the accommodation part 3a cannot be pressed and compressed.

When the accommodation part 3a is in the discharge state and the pressed state, in a case where the accommodation part 3a is switched to the non-discharge state and then switched to the non-pressed state, the accommodation part 3a is about to bulge outward due to the elastic recovery.

At this time, since the internal space of the accommodation part 3a is under negative pressure, the check valve 3c is opened to cause the space inside the accommodation part 3a, to communicate with the space inside the connection tube 3e and the space inside the transfer tube 2.

As a result, the exudate of the patient is suctioned into the drain 2a through each of the opening portions 2a3 to 2a5 of the drain 2a, and is further transferred into the inside of the accommodation part 3a through the transfer tube 2, the connection tube 3e, and the check valve 3c, and is accommodated in the accommodation part 3a.

The strength of the negative pressure (the suction force) of the suction pump 3 is preferably in a range of 20 to 30 mmHg and particularly preferably about 25 mmHg. However, there is no limitation thereto. 1 mmHg is about 133.3 Pa.

As described above, since the accommodation part 3a is formed to be transparent, it is possible to know, for example, the daily discharge amount of the exudate by comparing the scale 3al with the position of the liquid surface of the exudate accommodated in the accommodation part 3a from the outside of the suction pump 3. In addition, the color of the exudate accommodated in the accommodation part 3a can be viewed from the outside, and the situation inside the body can be estimated.

Even in a case where only a part of the accommodation part 3a is formed to be transparent instead of the entire accommodation part 3a, the discharge amount and the color of the exudate can be checked from the outside.

In addition, the shape of the accommodation part 3a is not particularly limited. For example, as shown in FIG. 1, the accommodation part 3a may be formed in a substantially elliptical or substantially circular shape in a front view, or may be formed in a substantially columnar or rectangular parallelepiped shape.

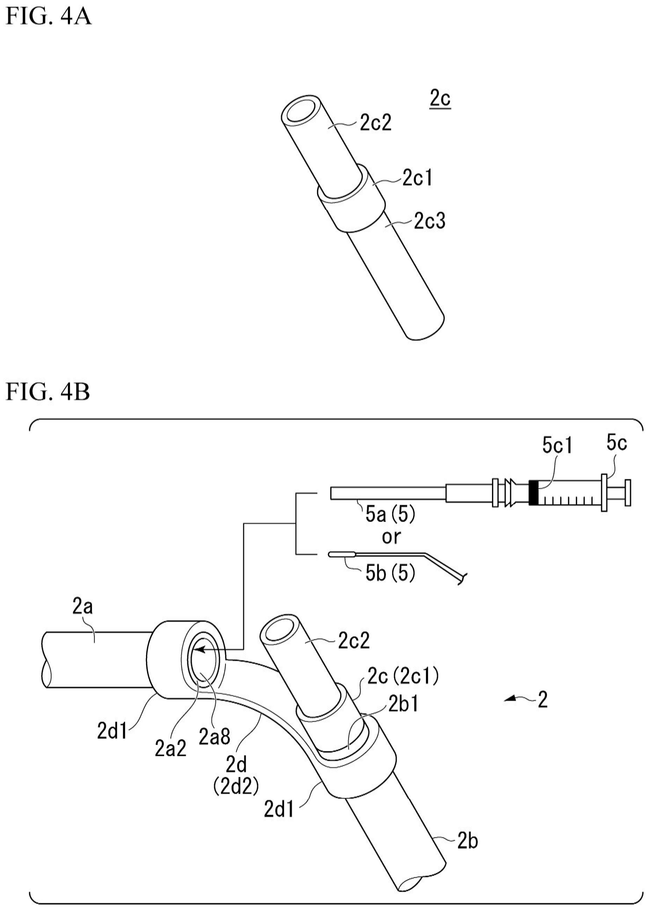

FIG. 4A is an overall perspective view of the joint 2c, and FIG. 4B is a schematic perspective view showing a state where the joint 2c is pulled out from the drain 2a and the connection between the drain 2a and the relay tube 2b is released.

As shown in FIG. 4A, the joint 2c is a hollow member having a large diameter portion 2c1 and two small diameter portions 2c2 and 2c3 each having a diameter smaller than the diameter of the large diameter portion 2c1 and being adjacent to the large diameter portion 2c1. The outer diameters of the two small diameter portions 2c2 and 2c3 are formed to be slightly larger than the inner diameters of the drain 2a and the relay tube 2b.

One small diameter portion 2c2 is inserted into the inside of the other end portion 2a2 of the drain 2a, and the other small diameter portion 2c3 is inserted into the inside of the relay tube 2b from one end portion 2b1, whereby the drain 2a and the relay tube 2b are connected to each other via the joint 2c to be capable of transferring the exudate.

The outer diameter of the joint 2c is preferably 2.7 mm (8 Fr in terms of outer circumference) or more in order to suppress the exudate from being clogged. However, there is no limitation thereto.

As shown in FIG. 4B, the connector 2d includes two substantially annular attachment portions 2d1 and a substantially strip-shaped connecting portion 2d2 that connects the two attachment portions 2d1. One attachment portion 2d1 is attached to the outer peripheral surface of the other end portion 2a2 of the drain 2a and the other attachment portion 2d1 is attached to the outer peripheral surface of one end portion 2b1 of the relay tube 2b, whereby the drain 2a and the relay tube 2b are connected to each other.

The length in a longitudinal direction of the connecting portion 2d2 is configured to be longer than the length of the small diameter portion 2c2 of the joint 2c in the extension direction (the direction in which the small diameter portion 2c2 extends).

With such a configuration, as shown in FIG. 4B, the small diameter portion 2c2 of the joint 2c can be pulled out from the drain 2a in a state where the drain 2a and the relay tube 2b are connected to each other by the connector 2d from the outer side, and the connection between the drain 2a and the relay tube 2b can be released. The connection as referred to herein refers to a connection between the end portions of the drain 2a and the relay tube 2b.

In the present embodiment, an opening portion 2a8 is formed in addition to one end portion 2a1 and the other end portion 2b2 which are the end portion openings of the transfer tube 2. The opening portion 2a8 is an example of a "first opening portion" that is accessible to the flow path 2i for the exudate, which connects one end portion 2a1 and the other end portion 2b2 of the transfer tube 2.

Therefore, by pulling out the small diameter portion 2c2 of the joint 2c from the drain 2a, the inside of the drain 2a can be easily cleaned by inserting a cleaning tool 5 into the inside (the flow path 2i) through the opening portion 2a8 of the other end portion 2a2 of the drain 2a, and clogging of the inside of the drain 2a due to the exudate or the like can be prevented or eliminated.

In addition, since the drain 2a and the relay tube 2b are connected by the connector 2d, when the joint 2c is pulled out, in a case where the drain 2a is gripped, it is possible to prevent the relay tube 2b from being unintentionally drooped.

The connector 2d is made of, for example, silicone rubber or polyvinyl chloride, so that the connector 2d can have sufficient durability to insert and remove the small diameter portion 2c2 of the joint 2c with respect to the drain 2a. However, the material of the connector 2d is not particularly limited.

The cleaning tool 5 may be any cleaning tool as long as it has a shape with an outer diameter narrower than the inner diameter of the drain 2a. For example, a rod-shaped brush 5b with a handle or an elongated rod-shaped cleaning tool such as a hollow tube 5a connected to a syringe 5c can be used.

In a case where the brush 5b is used, the brush 5b is inserted into the inside of the drain 2a from the other end portion 2a2 and is moved in and out to scrape out the inside of the drain 2a, whereby the exudate, the waste products, or the like clogged inside the drain 2a can be removed.

In addition, in a case where the tube 5a is used, after the tube 5a is inserted into the inside of the drain 2a from the other end portion 2a2, the exudate, the waste products, or the like clogged inside the drain 2a can be suctioned and removed by operating the syringe 5c to pull a piston 5c1. In the case of the 20 Fr drain 2a, the length of the outer circumference of the tube 5a is desirably about 8 Fr.

In a case where the cleaning tool 5 is inserted into the inside of the drain 2a from the other end portion 2a2, it is preferable that a protrusion portion protruding radially outward from the outer peripheral surface of the cleaning tool 5 is provided such that a tip part of the cleaning tool 5 does not enter the inside of the body and damage the tissue. In this way, since the excessive insertion of the cleaning tool 5 is restricted, even an operator who is not accustomed to handling the cleaning tool 5 can clean the inside of the drain 2a by using the cleaning tool 5 with confidence.

In addition, instead of providing the protrusion portion, a scale indicating the amount (length) of insertion into the drain 2a may be provided on the tube 5a. By inserting the tube 5a into the drain 2a while viewing the scale, it is possible to effectively suppress the tube 5a from entering the inside of the body through the opening portion 2a3 of the drain 2a.

After cleaning the inside of the drain 2a, the small diameter portion 2c2 of the joint 2c pulled out from the drain 2a before cleaning is inserted into the drain 2a again, so that the drain 2a and the relay tube 2b can be easily reconnected.

The wound treatment device 1 configured as described above can heal a wound of a patient by using the wound treatment device 1 as described below.

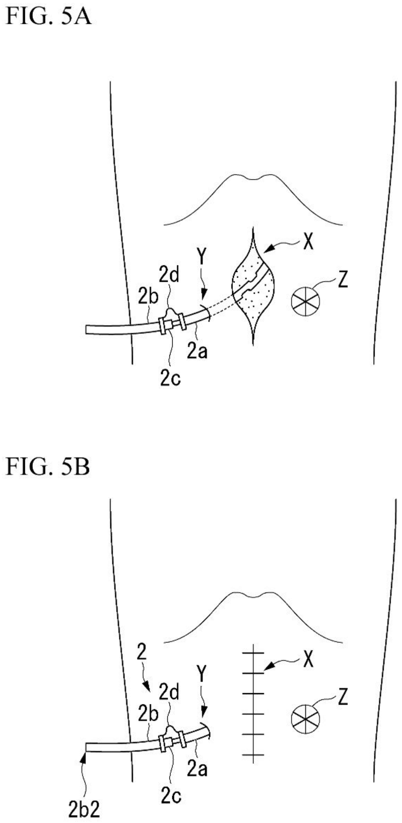

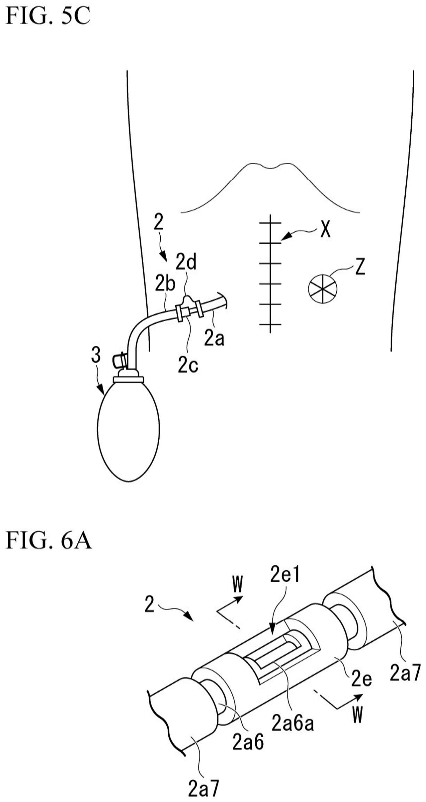

FIGS. 5A to 5C are schematic plan views showing a method for treating a wound part using the wound treatment device 1 shown in FIG. 1. FIG. 5A corresponds to an insertion step described in detail below, FIG. 5B corresponds to a closing step, and FIG. 5C corresponds to a connection step.

FIG. 5 shows an example of healing a postoperative wound dehiscence midline incision X of a patient who has undergone the construction of a colostomy as treatment for gastrointestinal perforation as an example of a wound part.

First, a small incision is made in the vicinity of the midline incision X formed in the vicinity of the colostomy Z to form a hole Y for inserting the drain 2a, and the drain 2a of the transfer tube 2 is passed through the hole Y from the one end portion 2a1 side to a position inside the midline incision X (insertion step, refer to FIG. 5A).

At this time, it is necessary to insert the drain 2a into the body to the other end portion 2a2 side located at a distance of several cm or more from the opening portion 2a5 located on the other end portion 2a2 side among the three opening portions 2a3 to 2a5 formed in the vicinity of one end portion 2a1.

In the case of the drain 2a of the present embodiment, since the opening portion 2a5 is formed from one end portion 2a1, which is a distal end, to a position of about 3 cm on the proximal side, the drain 2a is inserted into the body by 5 cm or more and at most about 20 cm from one end portion 2a1. In this way, the exudate can be reliably suctioned through the three opening portions 2a3 to 2a5.

Further, the drain 2a inserted into the body may be fixed to the tissue in the body with a medical adhesive, or may be sewn to the tissue using an absorbable suture that dissolves in the body. In this way, the drain 2a can be held at a place suitable for suction in the body.

In addition, it is desirable to detach the suction pump 3 from the transfer tube 2 in advance before the insertion step such that the suction pump 3 does not interfere with the surgery. However, the insertion step may be performed in a state where the transfer tube 2 is connected to the suction pump 3, and in this case, the connection step described later is not necessary.

In addition, it is desirable that the drain 2a is linearly extended in the body such that the exudate can be reliably suctioned.

In this way, in a case where one end portion 2a1 of the drain 2a is set below the midline incision X, the skin on the left and right sides of the midline incision X is sutured to close the midline incision X (closing step, refer to FIG. 5B).

Next, the relay tube 2b of the transfer tube 2 is connected to the connection tube 3e of the suction pump 3 (connection step).

After the connection tube 3e is connected to the suction pump 3, and after the discharge state and the pressed state are made as described above, the state is switched to the non-discharge state while maintaining the pressed state, and thereafter, the non-pressed state is made, so that the inside of the accommodation part 3a is made to be under negative pressure, and the transfer of the exudate into the accommodation part 3a through the transfer tube 2 is performed (transfer step).

In this way, the exudate or the waste products in the wound can be discharged to the accommodation part 3a outside the body through the transfer tube 2.

In addition, by making the inside of the accommodation part 3a negative pressure, it is possible to suppress the occurrence of retrograde infection to the inside of the body, and it is possible to promote the formation of granulation tissue by negative pressure.

During the transfers of the exudate, for example, once a day or at regular intervals, or when the inside of the drain 2a is clogged, as shown in FIG. 4B, the patient or the medical worker releases the connection between the drain 2a and the relay tube 2b and cleans the drain 2a by using the cleaning tool 5 (cleaning step).

In a wound healing device that is used in the negative pressure wound therapy of the related art, as the inside of a tubular member such as a hose for transferring exudate is further blocked, the excretion effect of the exudate decreases, and the possibility of retrograde infection or aggravation increases. Therefore, it has been a premise that the wound healing device is replaced in a case where the inside is further blocked.

On the other hand, in the present embodiment, since the connection between the drain 2a and the relay tube 2b can be released, it is possible to clean the inside of the drain 2a to prevent clogging inside the drain 2a, maintain the drainage effect, and suppress retrograde infection. In addition, even though the drain culture is positive, the drain culture can be made negative without replacing the drain 2a, and the drain 2a can be used without replacement until the wound heals.

In addition, since the replacement of the drain 2a is not required, it is possible to reduce the cost and the burden on the body of the patient.

In addition, when the exudate is accumulated in the accommodation part 3a of the suction pump 3, the exudate can be discharged from the discharge port 3b1a by making the pressed state after switching from the non-discharge state to the discharge state.

Finally, at a stage where the midline incision X is healed, the drain 2a is pulled out from the inside of the body (pulling-out step). The hole Y for inserting the drain 2a is naturally closed. According to the present treatment method using the wound treatment device 1, the midline incision X can be healed in a short period of about one week.

In fact, in a case where intestinal tract and skin, wound dehiscence of the midline incision, and surgical site infection occurred after the construction of a colostomy, it was extremely difficult to open the wound, wash the wound daily, and control infection, because the patient was elderly. Therefore, the wound was healed in about 3 weeks by re-suturing the intestinal tract and the skin and the midline incision by a reoperation, closing the skin by inserting the drain 2a into the body, and continuously suctioning the body fluid by using the suction pump 3.

In the treatment method of the related art, a treatment period in a range of 2 to 3 months including a period in a range of 1 to 2 weeks for a step of infection control such as washing of a wound part, a period in a range of 3 to 4 weeks for a step of negative pressure wound therapy using the wound treatment devices as described in Patent Documents 1 and 2, and a step of epithelialization is required, which is a great burden on patients.

On the other hand, in the treatment method using the wound treatment device 1 according to the present embodiment, unlike the negative pressure wound therapy in the treatment method of the related art, the exudate is continuously suctioned in a state where the skin is closed.

Therefore, the treatment method using the wound treatment device 1 according to the present embodiment can be used even for contaminated wounds and infected wounds, which have been contraindicated in the negative pressure wound therapy of the related art, and the process of epithelialization is not required, the treatment period can be significantly shortened, and the burden on the patient can be significantly reduced.

In addition, even in a case of being used for contaminated wounds, infected wounds, necrotic tissues, and the like, the wound can be naturally cured without the need for excising necrotic tissues by continuing drainage while closing the skin, thereby maintaining a wet environment and promoting phagocytosis. In the case of the colostomy, as a result of culturing the exudate (drainage fluid) transferred by the drain 2a in the third week of the treatment, the negative of the bacteria could be confirmed. The negative conversion of the drainage fluid of the drain culture could also be confirmed in clinical cases of wound dehiscence, bile leakage, and pancreatic fistula.

In addition, the wound treatment device 1 is configured as a manual pump, unlike the device used in the negative pressure wound therapy of the related art, and does not include an expensive member such as a vacuum pump, and each of the lid part 3d, the connection tube 3e, the check valve 3c, and the discharge tube 3b1 is formed of resin, the plug 3b2 is formed of a rubber material, the drain 2a and the accommodation part 3a are formed of silicone resin, and the relay tube is formed of polyvinyl chloride. Therefore, the wound treatment device 1 can be manufactured to be very lightweight and inexpensive, and hardly restricts the movement of the patient. In addition, the structure of the wound treatment device 1 can be simplified.

In addition, by forming the wound treatment device 1 with the non-metallic material in this way, it is possible to prevent the occurrence of halation in a case where an MRI examination or a CT examination is performed.

Further, in the case of the device that is used for the negative pressure wound therapy of the related art, the strength of the negative pressure is in a range of about 100 to 125 mmHg, and thus there is a risk of organ injury such as intestinal perforation in some cases. However, since the strength of the negative pressure of the suction pump 3, which is manual, is as small as about 25 mmHg, the suction pump 3 can be used with confidence.

In addition, in the case of the negative pressure wound therapy of the related art, it is necessary to replace a pad or a tape, such as a sponge, several times a week. However, according to the treatment method using the wound treatment device 1, there is no need to replace the pad or the tape, and the transfer step is performed for several days to several months depending on the healing of the wound. It is possible to reduce the burden on the patient.

The use of the wound treatment device 1 is not limited to the midline incision X described above, and the wound treatment device 1 can be used for treating various wound parts such as a wound part in which an organ is exposed, such as a treatment of a surgical site infection (SSI) of bile leakage, pancreatic fistula, infected wound, and contaminated wound after liver resection, a treatment of wound dehiscence, a prevention and treatment of mediastinitis in cardiac surgery, a treatment of an intra-abdominal abscess, a combined treatment in keloid excision, a wound of an artificial joint, a deep SSI treatment, and the like. In any case of the wound, one end portion 2a1 of the drain 2a is disposed at a position inside the wound in the body, and after the wound is closed, the wound can be healed early by regularly cleaning the drain 2a or cleaning the drain 2a when clogging occurs inside the drain 2a, during a transfer step of several days to several weeks depending on the degree or type of the wound.

For example, in a case of being used for the treatment of a pancreatic fistula, the treatment can heal by inserting the drain 2a into the body to close the wound, and suctioning the pancreatic juice leaked out in the body with the suction pump 3 to prevent postoperative bleeding due to a false aneurysm, suppress drain retrograde infection, and prevent and treat an intra-abdominal abscess.

On the other hand, with respect to necrotizing soft tissue infection (necrotizing fasciitis), the wound treatment device is not used because it may cause infection and sepsis aggravation.

FIGS. 6A to 6F are views showing the vicinity of a portion of the drain 2a of the wound treatment device 1 according to another preferred embodiment.

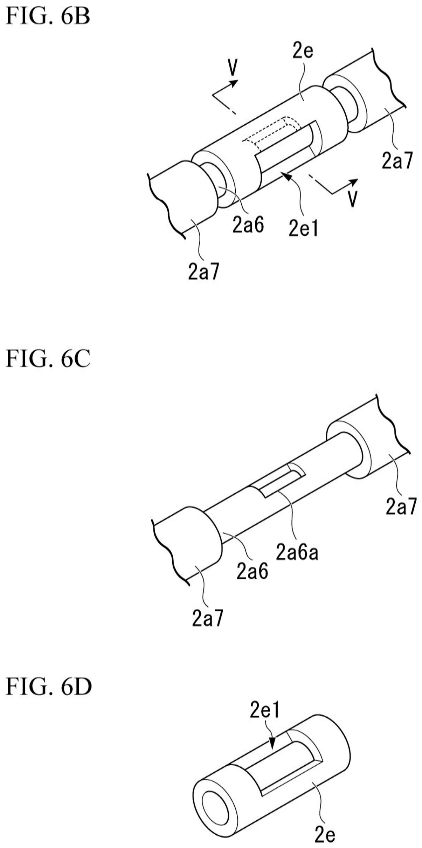

More specifically, FIG. 6A is a partially enlarged perspective view showing a state where the inside and the outside of the drain 2a communicate with each other, and FIG. 6B is a partially enlarged perspective view showing a state where the inside and the outside of the drain 2a shown in FIG. 6A are isolated from each other.

In addition, FIG. 6C is a partially enlarged perspective view of the drain 2a, and FIG. 6D is a perspective view of a first covering member 2e that covers the drain 2a.

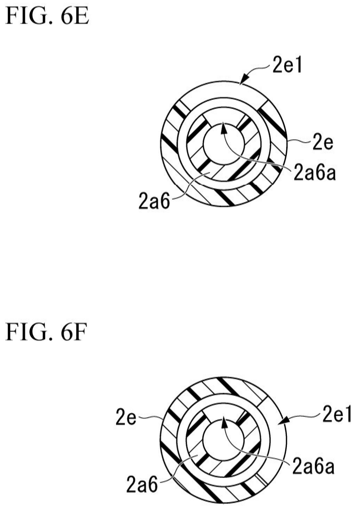

Further, FIG. 6E is a partial longitudinal sectional view taken along line W-W shown in FIG. 6A, and FIG. 6F is a partial longitudinal sectional view taken along line V-V shown in FIG. 6B.

The wound treatment device 1 according to the present embodiment is configured in the same manner as the wound treatment device 1 of the embodiment, except for the following points.

The transfer tube 2 according to the present embodiment is not provided with the connector 2d, the drain 2a and the relay tube 2b are connected to each other via the joint 2c, and the first covering member 2e described below is provided on the radially outer side of the drain 2a of the transfer tube 2.

In the present embodiment, the drain 2a includes a small diameter portion 2a6 and large diameter portions 2a7 that are located on both sides of the small diameter portion 2a6 and each has a diameter larger than the diameter of the small diameter portion 2a6, the small diameter portion 2a6 and the large diameter portions 2a7 being provided at a portion on the other end portion 2a2 side with respect to the center portion in the extension direction (the direction in which the drain extends, the direction of the flow path).

The first covering member 2e having a substantially tubular shape shown in FIG. 6D is rotatably provided on the radially outer side of the small diameter portion 2a6, and an outer peripheral surface of the small diameter portion 2a6 is covered by an inner peripheral surface of the first covering member 2e. In other words, the small diameter portion 2a6 passes through the inside of the substantially tubular first covering member 2e.

An opening portion 2a6a that radially penetrates to the inside of the drain (the flow path 2i) in a part of the small diameter portion 2a6 in the circumferential direction is formed in the small diameter portion 2a6.

Similarly, an opening portion 2e1 (a second opening portion) that radially penetrates to the inside of the first covering member in a part of the first covering member 2e in the circumferential direction is formed in the first covering member 2e.

With such a configuration, as shown in FIGS. 6A and 6E, when the positions of the opening portion 2a6a and the opening portion 2e1 overlap each other, the inside and the outside of the drain 2a communicate with each other through the opening portion 2a6a and the opening portion 2e1.

On the other hand, as shown in FIGS. 6B and 6F, when the positions of the opening portion 2a6a and the opening portion 2e1 do not overlap each other, the inside and the outside of the drain 2a do not communicate with each other through the opening portion 2a6a.

Therefore, only when the patient or the medical worker cleans the inside of the drain 2a with the cleaning tool 5, the first covering member 2e is rotationally operated in the circumferential direction such that the positions of the opening portion 2a6a and the opening portion 2e1 overlap each other, whereby the cleaning tool 5 can be inserted into the drain 2a through both the opening portions 2a6a and 2e1. The opening portion 2a6a is an example of a "first opening portion" that is accessible to the flow path 2i for the exudate, which connects one end portion 2a1 and the other end portion 2b2 of the transfer tube 2.

In addition, after the cleaning, the first covering member 2e is rotated again such that the positions of the opening portion 2a6a and the opening portion 2e1 are made different from each other, whereby the negative pressure is applied to the opening portions 2a3, 2a4, and 2a5 of the drain 2a, and the exudate can be suctioned.

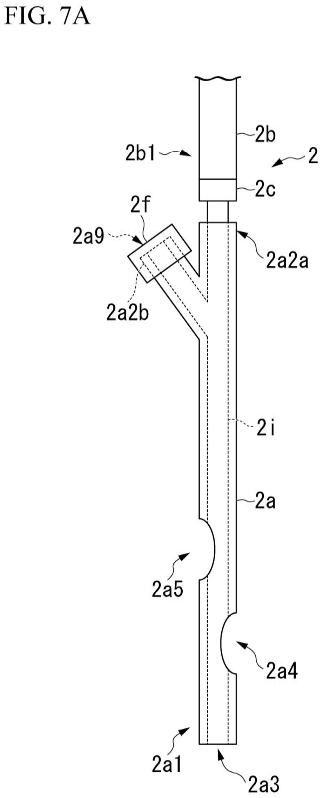

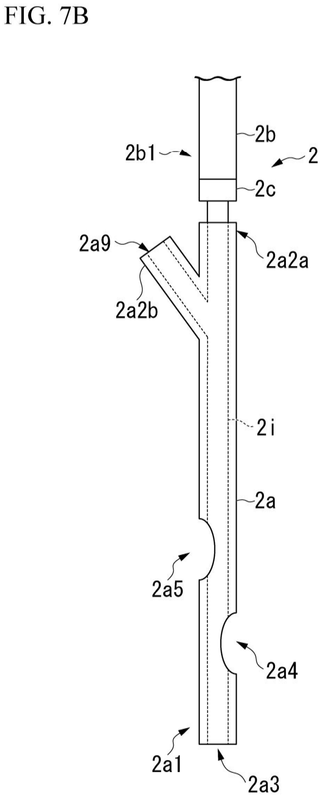

On the other hand, FIGS. 7A and 7B are schematic plan views showing the vicinity of the drain 2a of the wound treatment device 1 according to still another preferred embodiment.

FIG. 7A shows a state where a lid is attached to the other end portion of a branch from the drain 2a, and FIG. 7B shows a state where the lid is detached from the other end portion of the branch from the drain 2a.

In addition, in FIGS. 7A and 7B, the internal space of the drain 2a is shown by a broken line.

The wound treatment device 1 according to the present embodiment is configured in the same manner as the wound treatment device 1 according to the embodiment shown in FIGS. 1 to 5C, except for the following points.

The transfer tube 2 of the present embodiment is not provided with the connector 2d, and the drain 2a and the relay tube 2b are connected to each other via the joint 2c.

The drain 2a of the present embodiment has a substantially Y-shaped configuration that branches into two in a portion on the other end portion side with respect to the center portion in the extension direction (the direction in which the drain extends, the direction of the flow path), that is, in a portion close to the relay tube 2b.

One end portion 2b1 of the relay tube 2b is connected to one end portion 2a2a out of two end portions 2a2a and 2a2b branched on the other end portion side via the joint 2c. A lid 2f that covers an opening portion 2a9 formed in the end portion 2a2b is detachably attached to the other end portion 2a2b. The opening portion 2a9 is an example of a "first opening portion" that is accessible to the flow path 2i for the exudate, which connects one end portion 2a1 and the other end portion 2b2 of the transfer tube 2.

The drain 2a is inserted into the body of the patient from one end portion 2a1 at the time of surgery, and is inserted to a position before the branched portion, that is, on the one end portion 2a1 side with respect to the branched portion.

Therefore, the patient or the medical worker can access the flow path 2i and clean the drain 2a by detaching the lid 2f and inserting the cleaning tool 5 from the opening portion 2a9 of the other end portion 2a2b without releasing the connection between the drain 2a and the relay tube 2b. Then, after the cleaning is completed, the lid 2f is attached to the end portion 2a2b to block the opening portion 2a9, so that the negative pressure can be applied again.

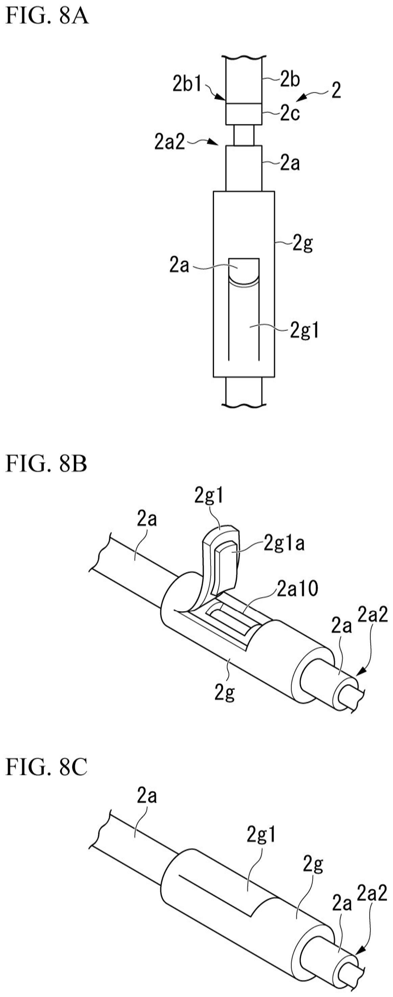

FIGS. 8A to 8C are enlarged views showing the vicinity of the drain 2a of the wound treatment device 1 according to another preferred embodiment.

More specifically, FIG. 8A is an enlarged plan view of the vicinity of the drain 2a and a second covering member 2g of the wound treatment device 1, FIG. 8B is an enlarged perspective view of the vicinity of the drain 2a showing a state where a turned-up portion of the second covering member 2g shown in FIG. 8A is greatly turned up, and FIG. 8C is an enlarged perspective view of the vicinity of the drain 2a showing a state where the turned-up portion of the second covering member 2g shown in FIG. 8A is attracted to the drain 2a.

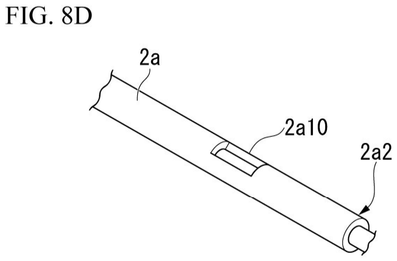

In addition, FIG. 8D is a partially enlarged perspective view of the drain 2a in a state where the second covering member 2g is detached.

The wound treatment device 1 according to the present embodiment is configured in the same manner as the wound treatment device 1 according to the embodiment shown in FIGS. 1 to 5C, except for the following points.

The transfer tube 2 of the present embodiment is not provided with the connector 2d, and the drain 2a and the relay tube 2b are connected to each other via the joint 2c.

The second covering member 2g having a substantially tubular shape that covers an outer peripheral surface of a portion of the drain 2a in the extension direction is provided on the radially outer side of the drain 2a of the transfer tube 2 of the present embodiment. In other words, the drain 2a of the transfer tube 2 passes through the inside of the substantially tubular second covering member 2g.

As shown in FIG. 8A, a cutout portion having a substantially inverted U-shape in a plan view is formed in the second covering member 2g, and thus a turned-up portion 2g1 that is turned up such that a part of the second covering member 2g is separated from the outer peripheral surface of the drain 2a is formed (refer to FIG. 8B).

An opening portion 2a10 that radially penetrates to the inside of the drain 2a (the flow path 2i) is formed in a portion of the drain 2a, which is covered with the turned-up portion 2g1. The patient or the medical worker can clean the inside of the drain 2a by inserting the cleaning tool 5 into the drain 2a through the opening portion 2a10, and can prevent or eliminate clogging. The opening portion 2a10 is an example of a "first opening portion" that is accessible to the flow path 2i for the exudate, which connects one end portion 2a1 and the other end portion 2b2 of the transfer tube 2.

On the other hand, a blocking piece 2g1a having substantially the same shape as the shape of the opening portion 2a10 (substantially rectangular parallelepiped shape) is provided on the inner surface of the turned-up portion 2g1 facing the opening portion 2a10.

The turned-up portion 2g1 is attracted to the opening portion 2a10 of the drain 2a as shown in FIG. 8C while the negative pressure is generated in the accommodation part 3a of the suction pump 3.

At this time, since the opening portion 2a10 is blocked by the blocking piece 2g1a, the transfer of the exudate can be performed without any problem even in a case where the opening portion 2a10 for cleaning is formed.

### [Wound Treatment Kit]

A wound treatment kit according to an embodiment includes the wound treatment device 1 and the cleaning tool 5 for cleaning the inside of the drain 2a of the wound treatment device 1.

As the wound treatment device 1 included in the wound treatment kit, the wound treatment device 1 according to each of the embodiments shown in FIGS. 1 to 8D can be used.

The cleaning tool 5 that is included in the wound treatment kit may be any cleaning tool as long as it has an outer diameter smaller than the inner diameter of the drain 2a, as described above (refer to FIG. 4B). For example, a rod-shaped brush 5b with a handle or an elongated rod-shaped cleaning tool such as a hollow tube 5a connected to the syringe 5c can be used.

The wound treatment kit may further include a transfer tube fixture described in detail below.

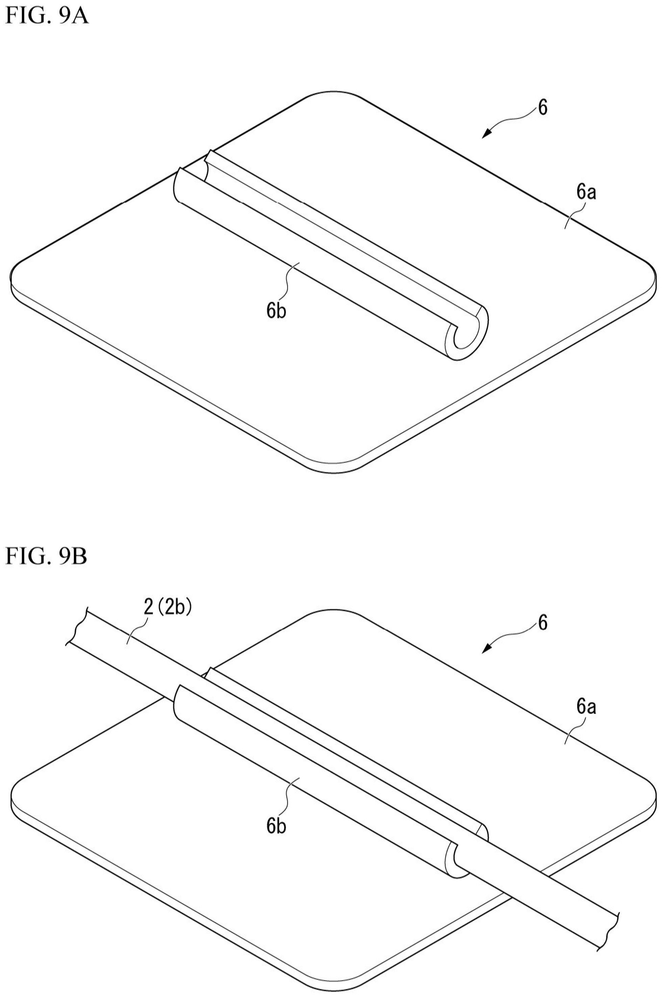

FIG. 9A is a schematic perspective view showing a transfer tube fixture that is included in the wound treatment kit, and FIG. 9B is a schematic perspective view showing a state where the transfer tube 2 shown in FIG. 9A is attached to the transfer tube fixture.

A transfer tube fixture 6 includes an adhesive pad 6a (an example of an adhesive part) having an adhesion layer on a lower surface thereof, and a fixing tube 6b (an example of a fixing part) attached to an upper surface of the adhesive pad 6a. The transfer tube fixture 6 can be attached to the skin of the patient by bringing the adhesion layer on the lower surface of the adhesive pad 6a into contact with the skin of the patient.

The fixing tube 6b is a member having flexibility and having a substantially C-shape in a longitudinal cross section. As shown in FIG. 9B, the transfer tube 2 can be fixed to the vicinity of the body surface of the patient by fitting the transfer tube 2 into the inside of the fixing tube 6b from above through an opening portion 6b1 formed in an upper portion of the fixing tube 6b. Therefore, the transfer tube 2 is difficult to hinder the movement of the patient.

In the related art, in a case where a tube such as a drain is fixed to a surface of the body of a patient, the tube is often attached to the body with a tape.

However, in a case where the drain 2a of the present embodiment is attached to the body with the tape, it is necessary to peel off the tape from the body each time the inside of the drain 2a is cleaned or the body is wiped, which is a heavy burden on the patient, such as skin irritation.

Therefore, the kit according to the present embodiment includes the transfer tube fixture 6, and the transfer tube 2 can be easily attached to and detached from the fixing tube 6b, so that the burden on the patient can be reduced. In other words, the fixing tube 6b can detachably fix the transfer tube 2.

The fixing tube 6b can be manufactured by cutting a hollow tube formed of a flexible material such as polyvinyl chloride in an extension direction of the tube.

Instead of the fixing tube 6b, a clip that clamps and fixes the transfer tube 2 may be fixed to the upper surface of the adhesive pad 6a, or other fixing means capable of detachably fixing the transfer tube 2 may be adopted as a fixing part.

### [Other Embodiments]

In one embodiment, the present invention provides a method for treating a wound part, the method including: a step of inserting a transfer tube of a wound treatment device, which includes the transfer tube for transferring exudate of a patient to the outside of the body, and a manual suction pump that is connected to the transfer tube and suctions the exudate to the inside thereof through the transfer tube, to a position inside the wound part through a hole formed in the vicinity of an open wound part; a step of closing the wound part into which the transfer tube is inserted; and a step of discharging the exudate inside the wound part to the outside of the body through the transfer tube by using the suction pump after the wound part is closed.

As the wound treatment device, the transfer tube, and the suction pump, those in the respective embodiments shown in FIGS. 1 to 10 can be used. The hole formed in the vicinity of the wound part corresponds to the hole Y described above.

In one embodiment, the present invention provides a method for treating a wound part, the method including a step of cleaning an inside of the transfer tube by using a cleaning tool having a shape narrower than an inner diameter of the transfer tube, between the steps of discharging the exudate inside the wound part to the outside of the body through the transfer tube by using the suction pump.

The present invention is not limited to each of the embodiments described above, and various modifications can be made within a scope of the invention described in the claims, and it goes without saying that those modifications are also included in the scope of the present invention.

For example, in each of the embodiments shown in FIGS. 1 to 9B, the accommodation part 3a is formed of an elastic material. However, the material of the accommodation part is not particularly limited. For example, in a case where the accommodation part is formed of a material having no elasticity, a member having a repulsive force, such as a spring, is accommodated inside the accommodation part, so that negative pressure can be generated inside the accommodation part by inflating the accommodation part by a repulsive force of a spring or the like when the state is switched from the pressed state to the non-pressed state. In other words, the accommodation part creates a negative pressure in the internal space by elastic recovery after deformation due to the external pressure. However, the elastic recovery after deformation of the accommodation part is not limited to that by the material itself of the accommodation part, and may be action by a spring or the like disposed inside the accommodation part.

In addition, in each of the embodiments shown in FIGS. 1 to 9B, the drain 2a and the relay tube 2b are connected to each other via the joint 2c. However, the drain 2a and the relay tube 2b may be configured to be directly connected to each other without the joint 2c by making the diameter of the drain 2a different from the diameter of the relay tube 2b. In this case as well, it is preferable that the connection between the drain 2a and the relay tube 2b can be released and can be reconnected. Further, it is preferable that the connector 2d is configured to be able to release the connection between the other end portion of the drain 2a and one end portion of the relay tube 2b while the drain 2a and the relay tube 2b are connected to each other.

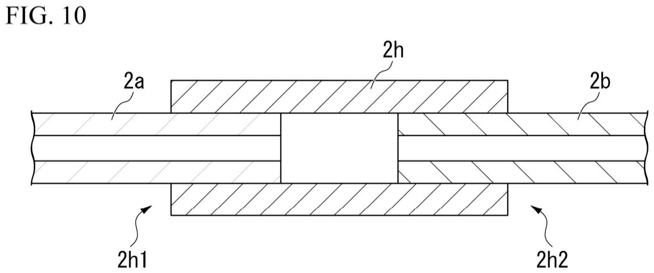

Further, in each of the embodiments shown in FIGS. 1 to 9B, the joint 2c is inserted into the inside of the drain 2a and the inside of the relay tube 2b, so that the drain 2a and the relay tube 2b are connected to each other. However, as shown in FIG. 10, the drain 2a and the relay tube 2b may be connected to each other to surround the drain 2a and the relay tube 2b by using a substantially tubular joint 2h.

FIG. 10 shows a sectional end view of the drain 2a and the relay tube 2b that are vertically cut along a surface extending in the extension direction of the drain 2a and the relay tube 2b.

In this case, the drain 2a is inserted into an opening portion 2h1 on one side of the joint 2h and the relay tube 2b is inserted into an opening portion 2h2 on the other side, so that the drain 2a and the relay tube 2b can be connected to each other.

By connecting the drain 2a and the relay tube 2b in this way, the inner diameter of the connection portion between the drain 2a and the relay tube 2b is not narrowed, and the clogging of the joint portion due to the exudate can be effectively suppressed.

In addition, in each of the embodiments shown in FIGS. 1 to 9B, the opening portions 2a4 and 2a5 that radially penetrate to the inside of the drain are formed on the outer peripheral surface of the drain 2a for the purpose of suction of the exudate. However, the number of opening portions for the purpose of suction of the exudate, which are formed on the outer peripheral surface, is not particularly limited.

However, in a case where the number of the opening portions for the suction of the exudate provided on the outer peripheral surface of the drain 2a is three or more, there is a concern that the suction force that is applied to each opening portion may be significantly reduced. Therefore, it is preferable that the number of opening portions on the outer peripheral surface is one or two. The dimensions of each opening portion are desirably 5 mm or more because the opening portion is soon blocked by the exudate or the like in a case where the dimensions are too small.

In addition, in each of the embodiments shown in FIGS. 6 to 10, the transfer tube 2 includes the drain 2a and the relay tube 2b. However, the transfer tube 2 does not need to necessarily include the relay tube 2b. Therefore, the flow path 2i of the transfer tube 2 may be formed only by the drain 2a by connecting the other end portion 2a2 of the drain 2a to the suction pump 3.

In this case, the flow path 2i of the transfer tube 2 is a flow path connecting one end portion 2a1 and the other end portion 2a2 of the drain 2a. In addition, each of the opening portions 2a6a, 2a9, and 2a10 of each embodiment in this case is an example of a "first opening portion" accessible to the flow path 2i for the exudate, other than the end portion openings 2a3 and 2a8 each formed at each of one end portion 2a1 and the other end portion 2a2 of the transfer tube 2.

In addition, in a case where the drain 2a is formed to be branched in a substantially Y-shape, as shown in FIG. 7, and the flow path 2i is formed only by the drain 2a, one end portion 2a2a out of the two branched end portions 2a2a and 2a2b is connected to the connection tube 3e of the suction pump 3.

### INDUSTRIAL APPLICABILITY

According to the present invention, since the wound treatment device can be manufactured to be lightweight and compact without requiring a power source by using a manual pump, and hardly restricts the movement of the patient, the wound treatment device can be used industrially.

### REFERENCE SIGNS LIST

1 Wound treatment device
2 Transfer tube
2a Drain
2b Relay tube
2c Joint
2d Connector
2e First covering member
2f Lid
2g Second covering member
3 Suction pump
3a Accommodation part
3b Discharge part
3b1 Discharge tube
3b1a Discharge port
3b2 Plug
3c Check valve
3d Lid part
3e Connection tube
5 Cleaning tool
5a Tube
5b Brush
5c Syringe
5c1 Piston
6 Transfer tube fixture
6a Adhesive pad
6b Fixing tube
X Midline incision
Y Hole
Z Colostomy

## Claims

1. A wound treatment device comprising:
a transfer tube that has one end portion which is inserted into a body of a patient and that is configured to transfer an exudate generated inside the body to an outside of the body; and
a manual suction pump that is connected to the other end portion of the transfer tube outside the body and that is configured to suction the exudate into an inside thereof through the transfer tube,
wherein the suction pump comprises
an accommodation part that creates a negative pressure in the internal space by elastic recovery after deformation due to external pressure, and that suctions and accommodates the exudate in the body into the internal space through the transfer tube,
a discharge part that is provided to be openable and closable and that is configured to discharge a fluid in the internal space of the accommodation part, and
a check valve that is provided at a connection location with the transfer tube and that is configured to prevent a backflow of the fluid from the internal space of the accommodation part to the transfer tube,
a flow path for the exudate is formed inside the transfer tube, connecting the one end portion and the other end portion, and
in addition to an end portion opening formed in each of the one end portion and the other end portion, a first opening portion accessible to the flow path is formed in the transfer tube.

2. The wound treatment device according to Claim 1, wherein the transfer tube comprises a drain having one end portion that is inserted into the body of the patient, and a relay tube that is configured to connect the drain and the suction pump, and
the drain is switchable between a state where the drain is connected to the relay tube and a state where the connection with the relay tube is released, and the first opening portion is formed in the other end portion of the drain, which is connected to the relay tube.

3. The wound treatment device according to Claim 2, wherein the transfer tube comprises a connector that is configured to connect the drain and the relay tube from an outside.

4. The wound treatment device according to Claim 1, wherein the transfer tube has a substantially Y-shaped configuration that branches into two at a portion on the other end portion side with respect to a center portion in an extension direction,
one end portion out of two branched end portions is connected to the suction pump, the first opening portion is formed at the other end portion, and a lid covering the first opening portion is detachably attached to the other end portion.

5. The wound treatment device according to Claim 1, wherein a substantially tubular covering member is provided on a radially outer side of the transfer tube to cover an outer peripheral surface of a portion of the transfer tube in an extension direction, and
the first opening portion radially penetrates the outer peripheral surface of a part of the transfer tube in a circumferential direction, which is covered with the covering member, to the flow path.

6. The wound treatment device according to Claim 5, wherein the covering member is rotatably provided on the radially outer side of the transfer tube, and
a second opening portion that radially penetrates to an inside of the covering member is formed in a part of the covering member in the circumferential direction.

7. The wound treatment device according to Claim 5, wherein a turned-up portion configured to be turned up away from the outer peripheral surface of the transfer tube is formed in the covering member, and
a blocking piece configured to block the first opening portion is provided on an inner surface of the turned-up portion facing the first opening portion.

8. The wound treatment device according to Claim 3, wherein a third opening portion that radially penetrates to the flow path is formed on an outer peripheral surface of the drain, and
an inner peripheral surface of the drain is circular.

9. A wound treatment kit comprising:
the wound treatment device according to any one of Claims 1 to 7; and
a cleaning tool for cleaning the flow path of the transfer tube.

10. The wound treatment kit according to Claim 9, wherein the cleaning tool is a rod-shaped brush.

11. The wound treatment kit according to Claim 9, wherein the cleaning tool is a hollow tube connected to a syringe, and
an outer diameter of the tube is smaller than an inner diameter of the transfer tube.

12. The wound treatment kit according to Claim 9, further comprising: a transfer tube fixture that comprises
an adhesive part that is attached to a skin of a patient, and
a fixing part that is attached to the adhesive part and that is configured to detachably fix the transfer tube.
